# EUROPEAN PATENT SPECIFICATION

(11) **EP 0 912 156 B1**
(45) Date of publication and mention of the grant of the patent: **29.08.2001**
(21) Application number: 97925064.4
(22) Date of filing: 05.06.1997
(51) Int. Cl.: A61K 7/00, A61K 7/32

(54) **COOLING EFFECT OF ANTIPERSPIRANT COMPOSITION**
KÜHLWIRKUNG EINER SCHWEISSHEMMENDEN ZUSAMMENSETZUNG
EFFET REFROIDISSANT DE COMPOSITION ANTISUDORIFIQUE

(30) Priority: 14.06.1996 GB 9612477
(43) Date of publication of application: 06.05.1999
(73) Proprietor: UNILEVER PLC, London EC4P 4BQ (GB); UNILEVER N.V., 3013 AL Rotterdam (NL)
(72) Inventor: MARTI, Vernon Peter John, Crosby, Liverpool, Merseyside L23 9UE (GB); TEMPLE, John, Crosby, Liverpool, Merseyside L23 9UE (GB)
(74) Representative: Pearce, Timothy
(86) International application number: EP9702983
(87) International publication number: WO9747273

(56) References cited:
- EP-A- 0 452 762
- GB-A- 2 273 299
- US-A- 4 806 338
- US-A- 5 082 652
- US-A- 5 160 732

## Description

The present invention relates to antiperspirant compositions. More particularly, the invention relates to aerosol antiperspirant compositions having improved cosmetic characteristics.

Antiperspirant and deodorant compositions can be applied to the skin by a variety of methods. Generally, such compositions comprise a carrier vehicle material in addition to an antiperspirant and/or deodorant active, the carrier and active being selected in accordance with factors such as the method of application, the intended use, the desired rheology and the desired cosmetic characteristics.

Aerosol compositions have gained wide consumer acceptance. Aerosol antiperspirant compositions generally comprise an anhydrous system comprising an antiperspirant salt dispersed in a liquid vehicle together with the liquified volatile propellant in a pressurised aerosol container.

Consumers can be divided into two classes - those who favour the use of antiperspirant aerosol compositions and those who favour deodorant aerosol compositions. Deodorant aerosol compositions do not contain significant amounts of antiperspirant active while the presence of the antiperspirant active in antiperspirant aerosol compositions imparts different cosmetic or sensory properties to the antiperspirant aerosol compositions. Generally, deodorant composition contain high levels (usually 50% or over) of alcohol, which has a deodorising effect and which imparts an exceptionally cold or fresh feeling to the deodorant composition.

Conversely, antiperspirant compositions are usually incompatible with high levels of alcohol and generally do not impart a sufficiently cold feeling.

Many consumers therefore choose deodorants over antiperspirants in order to achieve a cold or fresh feeling on application. However, deodorants unlike antiperspirants fail to prevent sweat generation and also exhibit poor malodour reduction over time.

Accordingly, consumers who initially select an aerosol deodorant over an antiperspirant for initial freshness fail to enjoy the long term benefits of an antiperspirant.

US 4,152,416 describes aerosol antiperspirant compositions capable of dispensing an astringent solid with low mistiness and dustiness. A polymer gum is used in the aerosol composition to reduce the mistiness and/or dustiness of the aerosol composition.

US 4,806,338 also describes the use of amino functional silicones in antiperspirant aerosol compositions in order to improve the cosmetic properties of the composition.

Moreover, US 4,806,338 implies that the use of such silicones helps to prevent undesirable cooling on the skin.

EP 343,843 of the Mennen Company also describes the use of a substantivity fluid made up of a silicone polymer dissolved in a carrier fluid to prevent clogging of aerosol valves at low delivery rates.

An object of the invention is to provide an aerosol antiperspirant composition as a skin cooling means which provides the sensory benefits of an aerosol deodorant.

According to the invention there is provided the use as a means of skin cooling of an antiperspirant aerosol composition comprising an antiperspirant active, a liquefied volatile propellant, a volatile cyclomethicone liquid carrier, and a silicone polymer that is a silicone gum or a silicone fluid, which silicone fluid has a viscosity of greater than 0.06 m²/s.

Preferably, the propellant has a boiling point between -45°C and +5°C.

The silicone polymer can be a silicone gum or a silicone fluid. Preferably, the silicone gum has a viscosity from 0.5 to 100 m²sec⁻¹ at 25°C.

Suitably, the silicone gum is a polydimethylsiloxane gum, preferably a dimethicone and/or dimethiconol gum.

The particulate antiperspirant material of the invention can be any of the known antiperspirant active materials. Particularly preferred materials are astringent metallic salts, in particular the inorganic and organic salts of aluminium, zirconium and zinc and mixtures thereof. Particularly preferred are the aluminium and zirconium salts such as aluminium halides, aluminium hydroxide halides, zirconium hydroxy halides, zirconium oxide halides and mixtures thereof. Generally, such aluminium and/or zirconium salts are any of those well known in the art. US patent no. 4,152,416 describes various aluminium zirconium salts which are suitable for use in the present invention. Typically, the antiperspirant active is present at from about 0.1% to about 20% by weight of the composition.

Generally, the silicone gums suitable for use in the present invention are as defined in US 4,152,416 and have a viscosity ranging from about 0.5 to 100 m²sec⁻¹ (500,000 to 100,000,000 centistokes) at 25°C. Typical silicone gums are the polydimethylsiloxane polymers such as dimethiconol and dimethicone gums.

The silicone gum is preferably present at levels from about 0.05% to about 6% by weight, more preferably from about 0.1% to about 4% by weight of the composition.

Silicone fluids can also be used

Suitable fluids are the DC200 series of silicones available from Dow Corning.

The volatile liquid carriers are the cyclomethicone liquids. Generally, the volatile low viscosity liquids usable in the present invention have a boiling point of at least 100LC and a viscosity of less than 1 x 10⁻⁵m²sec⁻¹ (10 centistokes) at 25°C. The volatile silicone fluids utilised at levels of about 1% to about 30%, preferably from about 2.5% to about 14.5% by weight of the composition.

Suitable silicone gums and volatile silicone fluids are available as standard proprietary material mixes or solutions e.g. Q21401 available from Dow Corning. SE30, a gum available from the General Electric Company can also be used.

The compositions contain one or more volatile aerosol propellant materials which in a gaseous state carry the other components of the invention in particulate or droplet form. Suitable propellants have a boiling point in the range of from -45°C to about 5°C and are present at levels from about 3.5% to 90% by weight of the composition.

Suitable aerosol propellants are well known in the art and include the chemically inert hydrocarbons such as propane, n-butane, isobutane, cyclopropane and mixtures thereof.

The antiperspirant compositions also comprise a suspending agent to suspend the antiperspirant actives. Suitable suspending agents include colloidal silicas and hydrophobic clays such as the bentonites. A particularly preferred bentonite is hydrophobic bentonite e.g. Bentone which is a bentonite treated with hydrophobic cationic materials. Typically the suspending agents are utilised at from about 0.3% to 3% by weight of the composition.

In addition, masking agents to conceal antiperspirant active suitable for use in the compositions can be included. Suitable masking agents are selected from aliphatic hydrocarbons (e.g. C8-C30, preferably C10-C16, more preferable C12-C15 linear or branched hydrocarbons), aliphatic esters, aromatic esters and mixtures thereof. The preferred residue masking agents for use in the compositions according to the present invention are C8-C30, preferably C10-C16, more preferably C12-C15 mono- and di-alkyl esters of aromatic carboxylic acids inclusive of the benzoates and phthalates. Suitable masking agents include isopropyl myristate, isopropyl palmitate, polydecenes, Fluid AP, the Finsolv range of benzoate esters and mixtures thereof which can be used at ranges from 0.5% to 25% by weight of the composition.

In addition to the abovementioned ingredients customary adjuncts of aerosol antiperspirant compositions can also be included in the composition. Such adjuncts include perfumes, bactericides, fungicides, emollients and other skin treating materials.

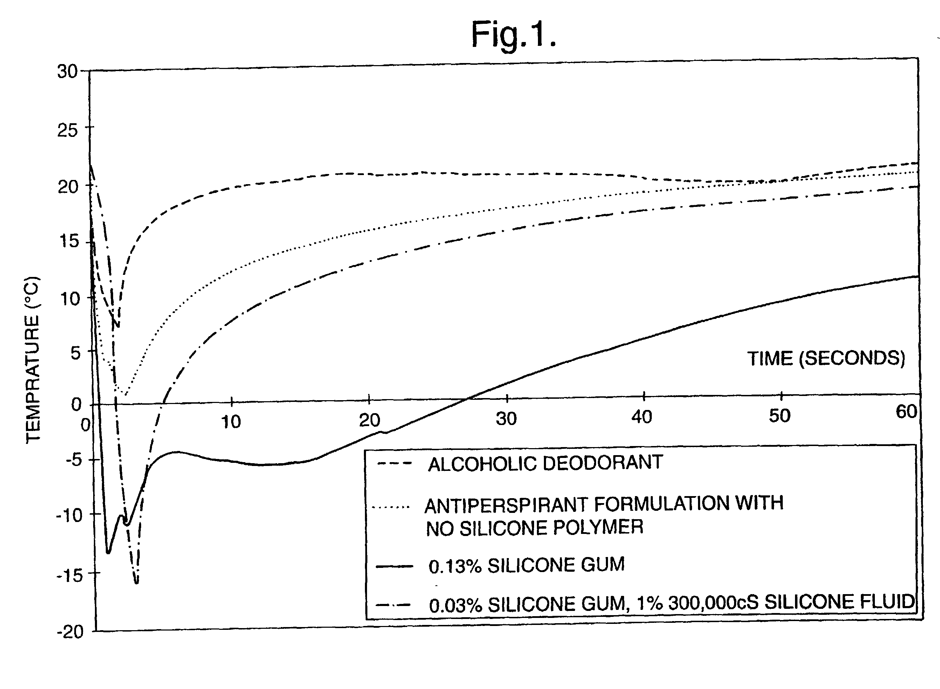

The following are examples of compositions used within the scope of the present invention. The invention is further illustrated by way of example only, with reference to figure 1, which shows a thermal imaging graph of recorded temperature over time with the spraying of various compositions. In the examples, all percentages of the specified ingredients are weight percentages.

### EXAMPLE 1

| Material | Chemical | Supplier | Level (%wt/wt) |
|---|---|---|---|
| AACH | Activated aluminium chlorohydrate | Giulini | 2.0 |
| Q2-1401 | Dimethiconol gum in cyclomethicone | Dow Corning | 1.0 |
| | Cyclomethicone | Dow Corning | 5.2 |
| Bentone 38 | Quatemium-18 hectorite | Rheox | 0.8 |
| | Perfume | | 1.0 |
| | Butane/Isobutane/ Propane | | 90.0 |

### EXAMPLE 2

| | |
|---|---|
| Activated aluminium chlorohydrate | 10% |
| Cyclomethicone | 5.0% |
| Fluid AP | 6.0% |
| Bentone 38 | 1.0% |
| Fragrance | 0.5% |
| Q2-1401 | 2.5% |
| Propellant | 75% |

### EXAMPLE 3

| | |
|---|---|
| Activated aluminium chlorohydrate | 1.0% |
| Cyclomethicone | 7.6% |
| Bentone 38 | 0.3% |
| Fragrance | 1.0% |
| SE 30 | 0.1% |
| Propellant | 90% |

### EXAMPLE 4

| | |
|---|---|
| Aluminium Chlorohydrate | 10% |
| DC 344* | 18.0% |
| Isopropyl palmitate | 6.0% |
| Q2-1401 | 1.0% |
| Bentone 38 | 1.5% |
| Ethanol | 10% |
| Fragrance | 0.7% |
| Talc | 2.8% |
| Propellant | 50% |

| | |
|---|---|
| *DC 344 is a cyclomethicone tetramer available from Dow Corning. | |

### EXAMPLE 5

| | |
|---|---|
| Activated aluminium chlorohydrate | 3.0% |
| DC 200 [60,000cS] | 1.0% |
| Cyclomethicone | 9.0% |
| Bentone 38 | 1.0% |
| Fragrance | 1.0% |
| Propellant | 85% |

The following comparative data illustrate the effect of the presence of a silicone gum on the cooling effect of an aerosol antiperspirant formulation as described in Example I.

### COMPARATIVE EXAMPLE 1

### 1. Thermal Imaging Data

The surface temperature of a patch of forearm skin was recorded using a calibrated Infra-red thermal imaging camera. The initial temperature recorded was 34.2°C.

Two aerosol products were sprayed onto the skin from a distance of 6 inches; a standard alcoholic deodorant made up of 50% propane, 0.5% perfume and 49.5% ethanol and a composition in accordance with Example 1. The temperature was recorded every ten seconds up to 90 seconds after application (Table 1). Table 1 clearly shows that for 1% Q2-1401, the skin was 4°C cooler following application than the alcohol deodorant and a temperature differential was maintained until approximately 30 seconds had elapsed.

Accordingly the data clearly demonstrates that by using antiperspirant aerosols of the invention a cooling effect similar to if not better than an alcoholic deodorant is obtained.

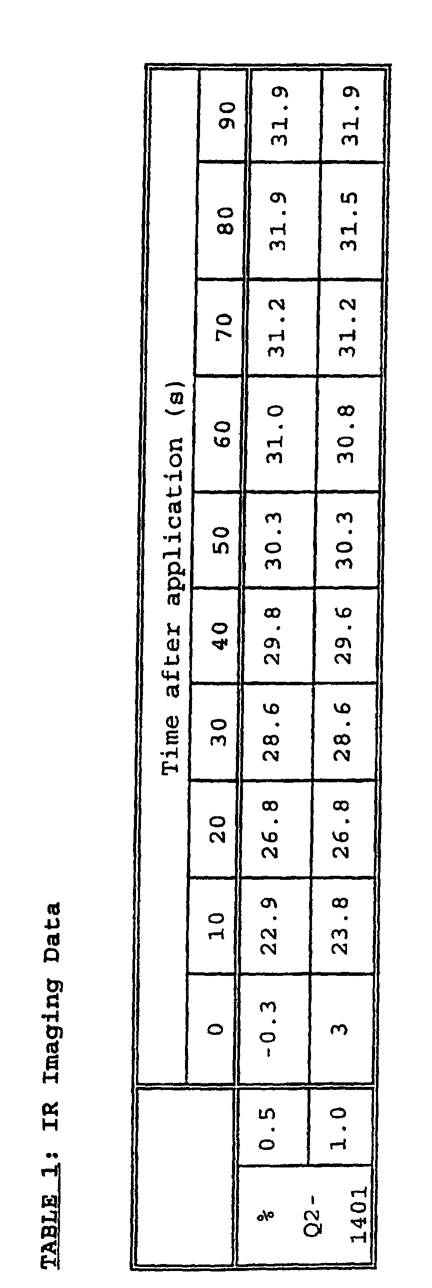

### 2. QDA Sensory data

A trained panel assessed the formulation of. Example I to compare same with an alcoholic deodorant. The panel was asked to describe the sensory properties of a product in terms of Quantitative Descriptor Analysis i.e for each descriptor to give a score from 0 to 100 on the intensity experienced. For two products to be significantly different on an attribute typically a 20 point difference was required. The results are presented in Table 2. It can be seen that;
1) both products were as cold
2) Example 1 was drier
3) Example 1 stung less
4) both products were equally adhesive
5) both products were as fresh
6) Example 1 was less wet
7) Both products were as tightening
8) Both the products gave equally low deposits

**TABLE 2 :**

| **QDA Sensory Data** | | |
|---|---|---|
| Attribute | Alcoholic deodorant | Example 1 |
| Cold on application | 68.1 | 66.2 |
| Wet on application | 43.4 | 21.7 |
| Stings on application | 36.1 | 12.9 |
| Sticky after application | 21.8 | 26.1 |
| Fresh after application | 55.9 | 68.1 |
| Wet after application | 40.7 | 18.7 |
| Tight after application | 13.3 | 21.9 |
| Deposit level after application | 8.5 | 17.3 |

### TABLE 3 : Deodorancy Test

This test was conducted on a panel of female subjects and results obtained after 24 hours. Each product was applied using a 2 second spray from a shaken can, 15.2 cm (6 inches)away from the axilla and deodorancy tested by a third party.

### The results were as follows:

| | Mean Malodour Score 24hr | Mean Dosage (mg) |
|---|---|---|
| Example 1 | 1.28 | 2010 |
| Example 1 without silicone | 1.39 | 1940 |
| Deodorant | 1.60 | 1950 |

| |
|---|
| Difference for significance @ 95% 0.14 |
| Difference for significance @ 99% 0.19 |

In this evaluation no significant difference in deodorancy efficacy was found between the two AACH-containing products and both of these were superior to the ethanolic deodorant after a 24 hour wear period at a 99% level of confidence.

Accordingly, the composition has the desirable sensory attributes of a deodorant (cooling and freshness), but benefits from antiperspirancy greater protection from malodour (>99% significantly less malodour than a deodorant after 24 hours) and is drier, and stings less.

Although the Applicants do not wish to be bound by any theory, it is believed that use of silicone gum in the antiperspirant compositions causes the propellant which is dissolved in the viscous gum to be conveyed to the skin surface. In prior art compositions propellant is not conveyed to the skin. Accordingly, in the present invention the propellant is volatilised by body heat at a slow rate thereby generating the desired deodorant type cooling effect.

### Comparative Example 2

### Thermal data

Three K-type thermocouples were arranged in a vertical line, and used to measure the temperature of various incident sprays. Temperature profiles were measured when various aerosols were sprayed at the thermocouple array for a period of 2 seconds , and from a distance of 2cm.

Four compositions were used; the first was an alcoholic deodorant containing 50% propellant, 0.5% perfume and 49.5% ethanol, and the second was an antiperspirant aerosol composition containing 10% antiperspirant active, 1.0% Bentone 38, 0.65% perfume, 13.35% cyclic silicone, and 75% propellant. The third composition was the same as Example 1 described above, whilst the fourth composition comprised 2% antiperspirant active, 0.2% Q2-1401, 1.0% 300,000cSt dimethicone, 5.1% cyclic silicone, 0.7% Bentone 38, 1.0% fragrance, and 90% propellant.

Spraying these formulations, it was found that the alcoholic deodorant composition produced an initial temperature drop from around room temperature (25°C) to 7°C. The second composition produced a temperature drop to 1°C. The third composition (containing silicone gum) produced a temperature drop to -13°C, whilst the fourth composition produced a temperature drop to -16°C.

The results are shown graphically in Figure 1. In this example, although the temperature drop recored for the alcoholic deodorant is actually less than for the antiperspirant compostion containing no silicone gums or fluids, in practice on application to the skin the alcoholic deodorant feels cooler than the second composition, since the ethanol in the alcoholic deodorant has a relatively high heat of vapourisation, and hence removes more heat from the skin when it vapourises. Also, alcohol will wet the skin more effectively than silicones. The remaining examples, all of which contain silicone gums or fluids, do not remove so much heat from the skin on application, since the heat of vaporisation of silicone is less than that of ethanol. As such, even though relatively low temperatures can be recorded by the thermocouple(e.g. -16°C), these compositions do not feel unpleasantly cold on application.

## Claims

1. Use as a means of skin cooling of an antiperspirant aerosol composition comprising an antiperspirant active, a liquefied volatile propellant, a volatile cyclomethicone liquid carrier, and a silicone polymer that is a silicone gum or a silicone fluid, which silicone fluid has a viscosity of greater than 0.06m²/s (60,000 cS).

2. The use as a means of skin cooling of an antiperspirant aerosol composition according to claim 1, **characterised in that** the propellant has a boiling point between -45°C and 5°C.

3. The use as a means of skin cooling of an antiperspirant aerosol composition according to claim 1 or claim 2, **characterised in that** the silicone polymer is a silicone gum.

4. The use as a means of skin cooling of an antiperspirant aerosol composition according to claim 1 or claim 2, **characterised in that** the silicone polymer is a silicone fluid.

5. The use as a means of skin cooling of an antiperspirant aerosol composition according to claim 3, **characterised in that** the gum has a viscosity from 0.5 to 100 m²sec⁻¹ at 25°C.

6. The use as a means of skin cooling of an antiperspirant aerosol composition according to claim 5, **characterised in that** the gum is a polydimethylsiloxane gum.

7. The use as a means of skin cooling of an antiperspirant aerosol composition according to claim 6, **characterised in that** the gum is a dimethicone and/or dimethiconol gum.

## Patentansprüche

1. Die Verwendung als ein Hautkühlmittel einer schweißhemmenden Aerosol-Zusammensetzung, enthaltend eine schweißhemmende aktive Verbindung, ein verflüssigtes flüchtiges Treibmittel, einen flüchtigen Cyclomethicon-Flüssigträger und ein Silicon-Polymeres, das ein Silicon-Gummi oder eine Silicon-Flüssigkeit ist, welche Silicon-Flüssigkeit eine Viskosität von größer als 0,06 m²/s (60,000 cSt) hat.

2. Die Verwendung als ein Hautkühlmittel einer schweißhemmenden Aerosol-Zusammensetzung nach Anspruch 1, **dadurch gekennzeichnet**, daß das Treibmittel einen Siedepunkt zwischen -45°C und 5°C hat.

3. Die Verwendung als ein Hautkühlmittel einer schweißhemmenden Aerosol-Zusammensetzung nach Anspruch 1 oder Anspruch 2, **dadurch gekennzeichnet**, daß das Silicon-Polymere ein Silicon-Gummi ist.

4. Die Verwendung als ein Hautkühlmittel einer schweißhemmenden Aerosol-Zusammensetzung nach Anspruch 1 oder Anspruch 2, **dadurch gekennzeichnet**, daß das Silicon-Polymere eine Silicon-Flüssigkeit ist.

5. Die Verwendung als ein Hautkühlmittel einer schweißhemmenden Aerosol-Zusammensetzung nach Anspruch 3, **dadurch gekennzeichnet**, daß das Gummi eine Viskosität von 0,5 bis 100 m²s⁻¹ bei 25°C hat.

6. Die Verwendung als ein Hautkühlmittel einer schweißhemmenden Aerosol-Zusammensetzung nach Anspruch 5, **dadurch gekennzeichnet**, daß das Gummi ein Polydimethylsiloxan-Gummi ist.

7. Die Verwendung als ein Hautkühlmittel einer schweißhemmenden Aerosol-Zusammensetzung nach Anspruch 6, **dadurch gekennzeichnet**, daß das Gummi ein Dimethicon- und/oder ein Dimethiconol-Gummi ist.

## Revendications

1. Utilisation comme moyen de refroidissement de la peau d'une composition antisudorifique en aérosol comprenant un actif antisudorifique, un propulseur volatil liquéfié, une cyclométhicone véhicule liquide volatile et un polymère de silicone qui est une gomme de silicone ou un fluide de silicone, ce fluide de silicone ayant une viscosité de plus 0,06 m²/s (60000 cSt).

2. Utilisation comme moyen de refroidissement de la peau d'une composition antisudorifique en aérosol selon la revendication 1, **caractérisée en ce que** le propulseur a un point d'ébullition entre -45 et 5°C.

3. Utilisation comme moyen de refroidissement de la peau d'une composition antisudorifique en aérosol selon la revendication 1 ou 2, **caractérisée en ce que** le polymère de silicone est une gomme de silicone.

4. Utilisation comme moyen de refroidissement de la peau d'une composition antisudorifique en aérosol selon la revendication 1 ou 2, **caractérisée en ce que** le polymère de silicone est un fluide de silicone.

5. Utilisation comme moyen de refroidissement de la peau d'une composition antisudorifique en aérosol selon la revendication 3, **caractérisée en ce que** la gomme a une viscosité de 0,5 à 100 m²s⁻¹ à 25°C.

6. Utilisation comme moyen de refroidissement de la peau d'une composition antisudorifique en aérosol selon la revendication 5, **caractérisée en ce que** la gomme est une gomme de polydiméthylsiloxane.

7. Utilisation comme moyen de refroidissement de la peau d'une composition antisudorifique en aérosol selon la revendication 6, **caractérisée en ce que** la gomme est une gomme de diméthicone et/ou de diméthiconol.
